# EUROPEAN PATENT SPECIFICATION

(11) **EP 0 441 160 B1**
(45) Date of publication and mention of the grant of the patent: **05.07.1995**
(21) Application number: 91100712.8
(22) Date of filing: 21.01.1991
(51) Int. Cl.: C07C 211/29, C07C 209/00, C07C 33/46, C12P 7/22

(54) **Process for preparing levo and dextro fenfluramine**
Verfahren zur Herstellung von links- und rechtsdrehendem Fenfluramin
Procédé pour la préparation de lévo- et dextrofenfluramine

(30) Priority: 09.02.1990 IT 1933190
(43) Date of publication of application: 14.08.1991
(73) Proprietor: LABORATORI MAG S.p.A., 20131 Milano (IT)
(72) Inventor: Magnone, Grato, I-20131 Milan (IT)
(74) Representative: Gervasi, Gemma, Dr.

(56) References cited:
- EP-A- 0 302 757
- DE-A- 2 245 467

## Description

This invention relates to a process for preparing levo and dextro fenfluramine via enantioselective reduction of 3-(trifluoro-methyl)-phenylacetone.

Fenfluramine [N-ethyl-α-methyl-3(trifluoromethyl)-benzene-ethanamine] is a known product [Chemical Abstract 59, 3831g (1963); Pinder et al Drugs 10, 241-325 (1975)], and has the following structural formula (1), were * indicates the optically active C atom: 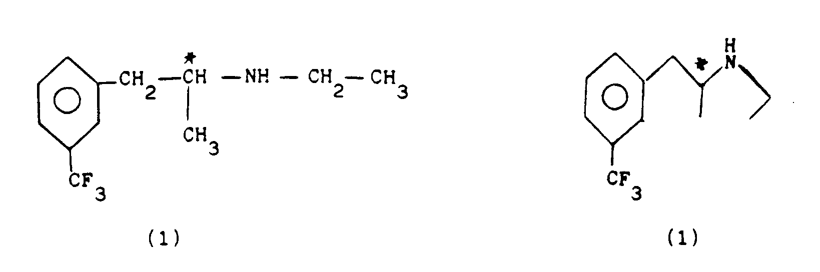
Fenfluramine is an anorexigenic drug widely used in that enantiomer form which rotates + 9,5° in ethanol, and known as dextrofenfluramine.

This product is obtained by classical optical resolution [Brit. Pat. 1,078,186 (1967) and French Pat. 1,517,587] starting from the raceme mixture, by firstly precipitating the levo salt with natural dibenzoyltartaric acid, in a diluted solution of ethanol or ethyl acetate, then recovering the base enriched with the dextrorotatory form, and finally obtaining this latter by crystallizing the salt with D-camphoric acid.

The need to arrive at the dextrorotatory form via double salification (dibenzoyltartaric acid and D-camphoric acid), the use of considerable solvent amounts and finally the impossibility of returning the unrequired enantiomer to the resolution cycle for racemization, make the final product very costly and of laborious preparation.

Owing to the above, a fenfluramine synthesis not based on classical optical resolution and leading directly to a single enantiomer was therefore considered to have a certain economical interest.

It has now been found that the ketone 1-(3-trifluoromethyl)-phenyl-propan-2-one, of structural formula (2), can be reduced by a fermentation path to give the corresponding optically active alcohol in that enantiomer form which rotates +24.8° in ethanol: 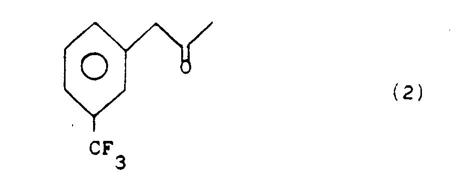
Each of the two alcohol enantiomer forms, which will be called (3) and ent-(3), is indicated schematically for ease of description by the following structural formulas; 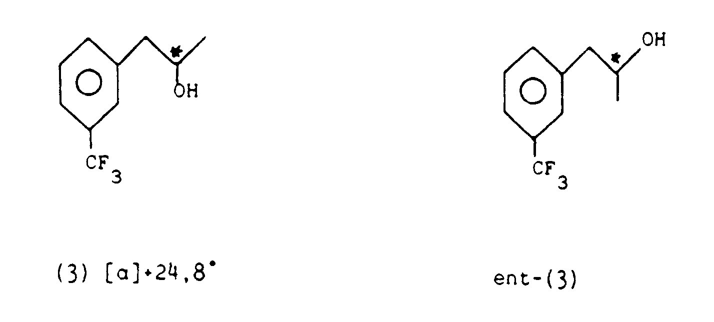
It is an object of the present invention to provide a method for synthesizing both the levo and dextro forms of fenfluramine based on the use, as key product, of 1-(3-trifluoromethyl)-phenyl-propan-2-ol in one of its two enantiomer forms (3) and ent-(3).

Another object of the invention is to provide 1-(3-trifluoromethyl)-phenyl-propan-2-ol in each of its two enantiomer forms.

1-(3-trifluoromethyl)-phenyl-propan-2-ol is described in DE-A-224 5467 in its racemic form.

The two enantiomer forms of the alcohol, (3) and ent-(3), can be obtained as follows.

The enantiomer form (3) is obtained by reducing the ketone (2) with yeast under fermentation and consists of only one enantiomer, within the accuracy of the NMR method used for analyzing the chirality.

The use of yeast under fermentation in the enantioselective reduction of carbonyl products is described in the literature [J.B. Jones, Tetrahedron 42, 3351 (1986)], but this specific case has never been described.

Particularly advantageous is the use of baker's yeast, particularly because of its low cost.

The ent-(3) form can be easily obtained enantiomerically pure starting from the enantiomer (3) by forming the corresponding tosylate in an environment made basic by an organic base, followed by configuration inversion of the optically active C atoms by substitution with benzoate (preferably sodium benzoate), and finally hydrolysis to give the ent-(3) enantiomer, the reaction conditions being easily optimized by the expert of the art. It is also possible to produce the (3) form from the ent-(3) form.

Tosyl chloride is preferably used for forming the tosyl derivative, and the organic base can be for example pyridine or Et₃N. 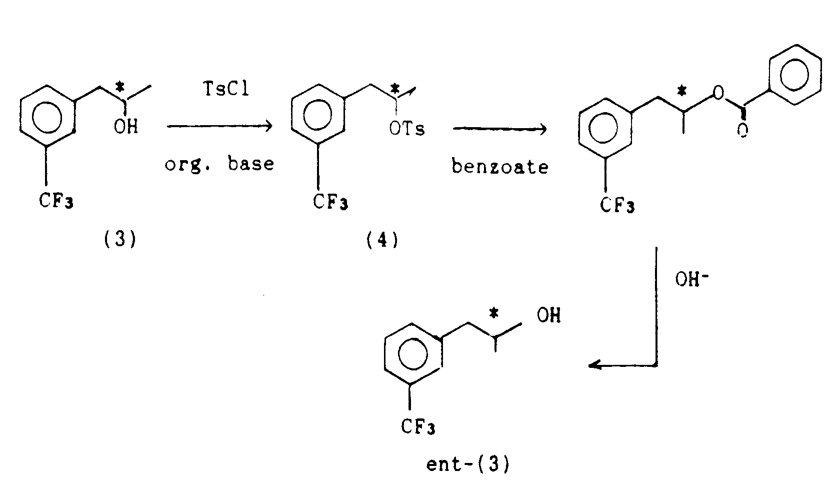
The dextro and levo forms of fenfluramine are each synthesized directly from ent-(3) and (3) respectively.

For example it is sufficient to transform each enantiomer of the alcohol into the corresponding tosyl derivative, ent-(4) or (4), react this with aside (preferably sodium aside in dimethylformamide at ambient temperature) to obtain the configuration inversion of the optically active C atom, reduce the azide to the amine ent-(7) or (7) (known as nor-fenfluramine) and reductively alkylate this latter, for example with acetaldehyde and sodium borohydride.

Again in this case the reaction conditions for these reactions are easily optimized by the expert of the art. 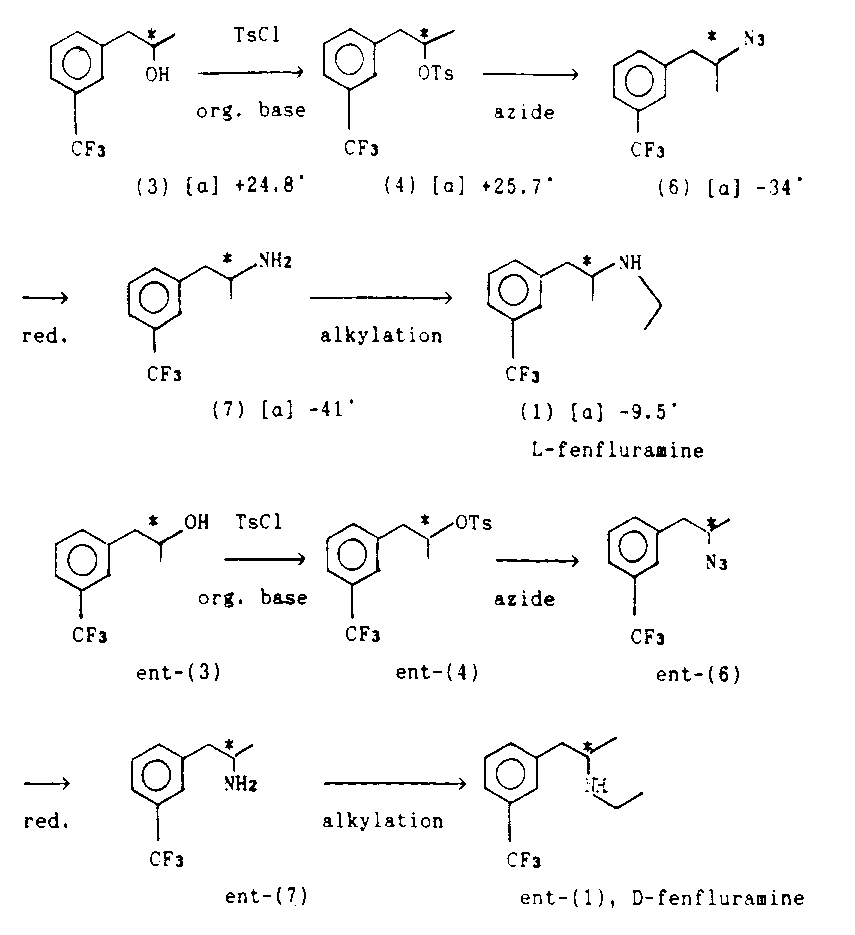
Also in this case, tosyl chloride and an organic base such as pyridine or Et₃N are preferred for the formation of the tosyl derivative.

The rotatory power of the two enantiomeric forms of fenfluramine, each obtained separately from the chiral alcohol resulting from the yeast reduction of the ketone (2) is equal in absolute value but of opposite sign and is in accordance with that described in the literature.

In this manner the two pure enantiomeric forms of fenfluramine are obtained by a simple procedure from an easily obtainable chiral product such as the alcohol (3).

From the above it follows that the preparation of L- and D-fenfluramine according to the present invention is extremely advantageous from the cost aspect for the following reasons:
- the synthesis steps for obtaining the optically active final product are extremely simple;
- low cost reagents are used;
- there is no loss or wastage of optically active products due to the total absence of racemics during the synthesis.
The synthesis of L- and D-fenfluramine is described hereinafter with reference to examples which are non-limitative of the scope of the invention.

### REDUCTION OF THE KETONE (2) TO (3) BY YEAST UNDER FERMENTATION

2.5 kg of baker's yeast (Distillerie Italiane) are dispersed in 10 litres of tap water at 25-30°C. 1 kg of D-glucose is added and the mixture stirred. After about 10 minutes CO₂ begins to evolve and at this point a solution of 50 ml of the ketone (2) in 50 ml of ethanol is dripped in over a period of 15 minutes. Stirring is continued for 16 hours, checking by tlc (thin layer chromatography, hexane/ethyl acetate 3:7) that reduction is complete at this point.

About 2 kg of celite or another equivalent diatomaceous earth and 2 litres of ethyl acetate are added and the mixture filtered through a wide Buchner filter containing a celite bed. The organic phase is separated, and extraction repeated with about 3 litres of ethyl acetate. The organic phases are gathered and dried and then evaporated. The residue is distilled at 70°C under 0.5-1 mmHg to give the alcohol (3). Yield 90%; [α]²⁰_{D} +24.8° (c 1, EtOH).

### CONVERSION OF THE ALCOHOL (3) INTO ENT-(3)

0.4 moles of alcohol (3) in 200 ml of methylene chloride at 0°C are treated with 0.8 moles of triethylamine and 0.6 moles of tosyl chloride.

After 12 hours at ambient temperature the reaction is complete.

The mixture is diluted with water and ice, and washed in sequence with water, dilute HCl solution, 3% wt/vol NaHCO₃ and water. It is dried over sodium sulphate, filtered and evaporated to dryness.

The tosylate (4) obtained in this manner (yield 88%) shows [α]²⁰_{D} +25.7° (c 1, EtOH).

This product, 0.3 moles, in 140 ml of dimethylformamide, is treated at boiling point under nitrogen with 0.45 moles of sodium benzoate for 5 hours. It is noted that after this period the reaction is complete. The reaction mixture is evaporated to dryness under vacuum, the residue taken up in water and boiled in the presence of an excess of 10% NaOH for 4 hours. The mixture is cooled, extracted with methylene chloride, the dried solution evaporated and the residue distilled under 1 mmHg to obtain at 70-75°C the alcohol ent-(3), [α]²⁰_{D} -23.8° (c 1, EtOH), with a yield of 78%.

### CONVERSION OF ENT-(3) INTO DEXTRO-FENFLURAMINE, ENT-(1)

0.3 moles of the alcohol ent-(3) are converted into the tosylate ent-(4), [α]²⁰_{D} -24.6°(c 1, EtOH), as indicated heretofore for obtaining (4) from (3). This material, 0.25 moles in 100 ml of dimethylformamide, is treated at a temperature ranging from 25 to 45°C (the reaction being slightly exothermic) with 0.5 moles of sodium aside under stirring.

The reaction is complete in 2 hours. The reaction mixture is poured into iced water containing a dilute 3% wt/vol solution of NaHCO₃, extracted with solvent three times (methylene chloride was used, but alternatively a 2:1 ethyl acetate/hexane mixture can be used).

The organic phase is washed a few times with water to remove the dimethylformamide, dried and evaporated. The residue consists of the azide ent-(6), [α]²⁰_{D} +34°(c 1, EtOH), with a yield of about 80% for the two steps.

The azide ent-(6), 0.2 moles in 70 ml of ethanol, is hydrogenated at atmospheric pressure in the presence of 10% Pd/C to give ent-(7) with quantitative yield, [α]²⁰_{D} +40° (c 1, EtOH). This material is dissolved in ethanol and 1.1 mol.eq. of acetaldehyde are added at ambient temperature. After 1 hour, 1 mol.eq. of NaBH₄ is added in portions, to obtain complete monoethylation.

The ethanol solution is concentrated, diluted with water, extracted with methylene chloride and distilled under high vacuum to obtain dextro-fenfluramine, ent-(1), with [α]²⁰_{D} +9.3° (c 8, EtOH), with a yield of 92% from the azide ent-(6).

### CONVERSION OF (3) INTO LEVO-FENFLURAMINE (1)

The aforedescribed sequence is repeated exactly, starting with (3). The levo-fenfluramine (1) obtained in this manner shows [α]²⁰_{D} -9.5°(c 8, EtOH).

## Claims

1. A process for preparing levo- and dextro-fenfluramine, characterised in that 1-(3-trifluoromethyl)-phenyl-propan-2-ol is used in one of its enantiomerically pure forms.

2. A process as claimed in claim 1, characterised in that the 1-(3-trifluoromethyl)-phenyl-propan-2-ol in one of its enantiomerically pure form is reacted with tosylate in an environment made basic by an organic base, then treated with azide and reduced to obtain nor-fenfluramine, which is then reductively alkylated to give enantiomerically pure fenfluramine.

3. A process as claimed in claim 2, wherein the tosylate is tosyl chloride and the organic base is chosen from Et₃N and pyridine.

4. A process as claimed in claim 2, wherein the reaction with azide is conducted in dimethylformamide at ambient temperature using sodium azide.

5. A process as claimed in claim 2, wherein the reductive alkylation is conducted with acetaldehyde and sodium borohydride.

6. A process for preparing 1-(3-trifluoromethyl)-phenyl-propan-2-ol in enantiomerically pure form, characterized in that 1-(3-trifluoromethyl)-phenyl-propan-2-one is subjected to enantioselective reduction by yeast under fermentation.

7. A process as claimed in claim 6, wherein baker's yeast is used.

8. A process for transforming enantiomerically pure 1-(3-trifluoromethyl)-phenyl-propan-2-ol into the corresponding enantiomer with configuration inversion of the optically active C atom, characterised in that the starting enantiomer is trasformed into the corresponding tosyl derivative in an environment made basic by an organic base, this is then treated with benzoate and finally hydrolyzed to give the corresponding enantiomer.

9. A process as claimed in claim 8, wherein tosyl chloride is used to obtain the tosyl derivative, and the organic base is chosen from Et₃N and pyridine.

10. A process as claimed in claim 8, wherein sodium benzoate is used.

11. 1-(3-trifluoromethyl)-phenyl-propan-2-ol in its enantiomeric form (3).

12. 1-(3-trifluoromethyl)-phenyl-propan-2-ol in its enantiomeric form ent-(3).

## Patentansprüche

1. Verfahren zur Herstellung von linksdrehendem und rechtsdrehendem Fenfluramin,
dadurch **gekennzeichnet**, daß
1-(3-Trifluormethyl)-phenylpropan-2-ol in einer seiner enantiomeren reinen Formen verwendet wird.

2. Verfahren nach Anspruch 1,
dadurch **gekennzeichnet,** daß
das 1-(3-Trifluoromethyl)-phenylpropan-2-ol in einer seiner enantiomeren reinen Formen mit Tosylat in einer Umgebung zur Reaktion gebracht wird, die durch eine organische Base basisch gemacht ist, dann mit Azid behandelt und reduziert wird, unter Erhalt von Nor-Fenfluramin, das dann reduktiv alkyliert wird, unter Erhalt von enantiomer reinem Fenfluramin.

3. Verfahren nach Anspruch 2,
worin das Tosylat Tosylchlorid ist und die organische Base aus Et₃N und Pyridin ausgewählt wird.

4. Verfahren nach Anspruch 2,
worin die Reaktion mit Azid in Dimethylformamid bei Umgebungstemperatur unter Verwendung von Natriumazid durchgeführt wird.

5. Verfahren nach Anspruch 2,
worin die reduktive Alkylierung mit Azedaldehyd und Natriumborhydrid durchgeführt wird.

6. Verfahren zur Herstellung von 1-(3-Trifluoromethyl)-phenylpropan-2-ol in enantiomerer reiner Form,
dadurch **gekennzeichnet,** daß
1-(3-Trifluoromethyl)-phenylpropan-2-on einer enantioselektiven Reduktion durch Hefe unter Fermentation unterworfen wird.

7. Verfahren nach Anspruch 6,
worin Bäckerhefe verwendet wird.

8. Verfahren zum Transformieren von enantiomer reinem 1-(3-Trifluoromethyl)-phenylpropan-2-ol in das entsprechende Enantiomer unter Konfigurationsumwandlung des optisch aktiven C-Atoms,
dadurch **gekennzeichnet,** daß
das Ausgangsenantiomer in das korrespondierende Tosylderivat in einer Umgebung transformiert wird, die durch eine organische Base basisch gemacht ist, daß dieses dann mit Benzoat behandelt und schließlich hydrolysiert wird, unter Erhalt des entsprechenden Enantiomers.

9. Verfahren nach Anspruch 8,
worin Tosylchlorid verwendet wird, unter Erhalt des Tosylderivates, und worin die organische Base aus Et₃N und Pyridin ausgewählt wird.

10. Verfahren nach Anspruch 8,
worin Natriumzbenzoat verwendet wird.

11. 1-(3-Trifluoromethyl)-phenylpropan-2-ol in seiner enantiomeren Form (3).

12. 1-(3-Trifluoromethyl)-phenylpropan-2-ol in seiner enantiomeren Form ent-(3).

## Revendications

1. Procédé pour préparer la levo- et la dextro-fenfluramine, caractérisé en ce que l'on utilise le 1-(3-trifluorométhyl)-phényl-propan-2-ol dans l'une de ses formes énantiomériquement pures.

2. Procédé selon la revendication 1, caractérisé en ce que l'on fait réagir le 1-(3-trifluorométhyl)-phényl-propan-2-ol dans l'une de ses formes énantiomériquement pures avec un tosylate dans un environnement rendu basique par une base organique, on traite ensuite avec un azide et on réduit pour obtenir la norfenfluramine, qui est ensuite soumise à une alkylation réductive pour donner la fenfluramine énantiomériquement pure.

3. Procédé selon la revendication 2, caractérisé en ce que le tosylate est le chlorure de tosyle et la base organique est choisie parmi Et₃N et la pyridine.

4. Procédé selon la revendication 2, caractérisé en ce que la réaction avec l'azide est effectuée dans le diméthylformamide à température ambiante en utilisant l'azide de sodium.

5. Procédé selon la revendication 2, caractérisé en ce que l'alkylation réductive est effectuée à l'aide d'acétaldéhyde et de borohydrure de sodium.

6. Procédé pour la préparation de 1-(3-trifluorométhyl)-phényl-propan-2-ol dans une forme énantiomériquement pure, caractérisé en ce que la 1-(3-trifluorométhyl)-phényl-propan-2-one est soumise à une réduction énantiosélective par une levure en fermentation.

7. Procédé selon la revendication 6, caractérisé en ce que l'on utilise de la levure de boulanger.

8. Procédé pour transformer un 1-(3-trifluorométhyl)-phényl-propan-2-ol énantiomériquement pur en l'énantiomère correspondant ayant une inversion de configuration de l'atome de carbone optiquement actif, caractérisé en ce que l'énantiomère de départ est transformé en dérivé de tosyle correspondant dans un environnement rendu basique par une base organique, puis traité par un benzoate, et finalement hydrolysé pour donner l'énantiomère correspondant.

9. Procédé selon la revendication 8, caractérisé en ce que l'on utilise le chlorure de tosyle pour obtenir un dérivé de tosyle, et en ce que la base organique est choisie parmi Et₃N et la pyridine.

10. Procédé selon la revendication 8, caractérisé en ce que l'on utilise du benzoate de sodium.

11. 1-(3-trifluorométhyl)-phényl-propan-2-ol dans sa forme énantiomérique (3).

12. 1-(3-trifluorométhyl)-phényl-propan-2-ol dans sa forme énantiomérique ent-(3).
